(19) Europäisches Patentamt
European Patent Office
Office européen des brevets

(11) Publication number: **0 496 168 A1**

(12) **EUROPEAN PATENT APPLICATION**

(21) Application number: **91312022.6**

(51) Int. Cl.[5]: **C07D 233/32**, C08K 5/3445

(22) Date of filing: **24.12.91**

(30) Priority: **25.01.91 GB 9101690**

(43) Date of publication of application: **29.07.92 Bulletin 92/31**

(84) Designated Contracting States: **BE DE FR GB IT NL**

(71) Applicant: **BP Chemicals Limited**
**Belgrave House 76 Buckingham Palace Road**
**London, SW1W 0SU(GB)**

(72) Inventor: **Cracknell, Robert Brian, BP Chemicals Limited**
**Salt End, Hedon**
**Hull, HU12 8DS(GB)**

(74) Representative: **Perkins, Nicholas David et al**
**BP International Limited Patents and Agreements Division, Chertsey Road**
**Sunbury-on-Thames, Middlesex TW16 7LN(GB)**

(54) **Process for the production of N-(2-hydroxyethyl)-2-imidazolidinone.**

(57) A process for the production of N-(2-hydroxyethyl)-2-imidazolidinone by heating (a) monoethanolamine and carbon dioxide; (b) the N-(2-hydroxyethyl) carbamate salt of monoethanolamine; (c) 2-oxazolidinone and monoethanolamine or (d) N,N'-bis(2-hydroxyethyl) urea is catalysed by the presence of a base, preferably potassium or sodium carbonate.

EP 0 496 168 A1

The present invention relates to a process for the production of N-(2-hydroxyethyl)-2-imidazolidinone.

Processes for the production of N-(2-hydroxyethyl)-2-imidazolidinone from monoethanolamine and carbon dioxide are known.

Thus, US patent number US 2,812,333 describes a process for the preparation of N-(2-hydroxyethyl) ethylene diamine which comprises reacting 2-aminoethanol, or an aqueous solution thereof, with carbon dioxide to form 1-(2-hydroxyethyl)imidazolidinone-2 which is subsequently hydrolysed to N-(2-hydroxyethyl)ethylene diamine. The hydroysis is reversible. Thus, in Example IV of US 2,812,333 N-(2-hydroxyethyl)ethylene diamine is shown to react with carbon dioxide to produce 1-(2-hydroxyethyl) imidazolidone-2 by heating a 20% solution of N-(2-hydroxyethyl) ethylene diamine in the form of its carbonate salt for 5 hours at 175° C in a sealed autoclave at 32.4 barg (470 psig).

Also, French patent publication number FR 1,238,352 describes a process for the preparation of N-$\beta$-hydroxyethylimidazolidone and N-$\beta$-hydroxyethylenediamine by reacting carbon dioxide with 2-aminoethanol in the presence of water, characterised in that the reaction is performed at a temperature of at least 200° C and a pressure of at least 50 atmospheres in the presence of a quantity of water up to and including the weight of aminoethanol.

In the examples described in US 2,812,333 the highest conversion of a 20% solution of 2-aminoethanol to 1-(2-hydroxyethyl)imidazolidine-2 was only 25% in Example 10, this being achieved at 165° C, 41.4 barg (600 psig) initial pressure, after 5 hours heating. In FR 1,238,352 the highest conversion of aminoethanol was 64% with a selectivity to N-$\beta$-hydroxyethylimidazolidone of 85.5% of theoretical, this being achieved in 20 minutes but this was only achieved at extreme conditions that is at 240° C and 180 atmospheres.

The technical problem to be solved, therefore, is to provide a process for the production of N-(2-hydroxyethyl)-2-imidazolidinone which achieves high conversion of monoethanolamine and high product selectivity.

Thus according to the present invention there is provided a process for the production of N-(2-hydroxyethyl)-2-imidazolidinone which comprises contacting at elevated temperature, a base with one or more of (a) monoethanolamine and carbon dioxide; (b) the N-(2-hydroxyethyl) carbamate salt of monoethanolamine; (c) 2-oxazolidinone and monoethanolamine; and (d) N,N'-bis(2-hydroxyethyl)urea.

The present invention solves the problem defined above by the use of a base.

Preferably, the base comprises a carbonate, bicarbonate or hydroxide salt, more preferably a carbonate salt of an alkali metal or of an alkaline earth metal, and most preferably potassium or sodium carbonate. An effective amount of base is suitably about 0.5% to 30% by weight ratio to the amount of the other reactants. The amount of base required will depend upon the particular base, reagents, temperature and pressure used.

The carbamate salt may be prepared by contacting mono ethanolamine with carbon dioxide at a suitable temperature and pressure, for example at a temperature of about 25° C to 240° C and at an elevated pressure, for example of about atmospheric to 150 barg. The reaction may take place in neat reagents or in any suitable polar solvent.

The N,N'-bis(2-hydroxyethyl)urea may be prepared by reaction of 2-oxazolidinone with monoethanolamine at elevated temperature by known methods, for example as described by Balyasnikova, L.V et al Trudy Gosudarstvennyi nauchno-Issledovatel'skii 1 proektnyi Institut azotnoi promyshlennosh 1 produktov organicheskogo Sinteza 1977 44 37.

The 2-oxazolidinone for use in the process of the present invention or for the preparation of N,N'-bis (2-hydroxyethyl) urea is available commercially and may be prepared for example by reaction of monoethanolamine with carbon dioxide by known methods, for example as described by Bogolovski, Y.N. et al Referativnyi Zhumai Khimiia 1973 (Abst. No. 13G227).

The N-(2-hydroxyethyl)carbamate salt of monoethanolamine, the 2-oxazolidinone and the N,N'-bis(2-hydroxyethyl)urea may be prepared in situ by reaction of monoethanolamine with carbon dioxide.

In the process of the present invention, the elevated temperature is suitably at least about 120° C, preferably at least about 160° C. In the process of the present invention, the elevated temperature is suitably less than about 350° C, preferably less than about 250° C, more preferably less than 200° C and most preferably the elevated temperature is about 180° C.

The process of the present invention may be carried out at a total pressure of at least about 5 barg and of less than 140 barg, preferably at a pressure of about 20 to 100 barg.

Carbon dioxide is present for the reaction (a) of monoethanol amine. Carbon dioxide may also be present for the formation in situ of (b) the N-(2-hydroxyethyl) carbamate salt of monoethanolamine, (c) the 2-oxazolidinone and (d) the N,N' - bis (2-hydroxyethyl) urea. In which cases carbon dioxide should be present in at least stoichiometric amounts with respect to the other reactants. Carbon dioxide may also be present in the process of the present invention to reduce breakdown of the N-(2-hydroxyethyl) carbamate

salt of monoethanololamine, the 2-oxazolidinone and/or the N,N' - bis (2-hydroxyethyl) urea. Carbon dioxide is available commercially for use in the present invention and may be used with or without further purification. The carbon dioxide may be diluted with a gas which is inert under the reaction conditions, for example nitrogen or hydrogen. The partial pressure of carbon dioxide is suitably in the range 20 to 60 barg.

The process of the present invention may be performed in neat reactants or in a suitable solvent for example a polar solvent such as methanol.

The process of the present invention may be performed as a batch or continuous process.

The monoethanolamine and carbon dioxide; N-(2-hydroxyethyl) carbamate salt of monoethanolamine; 2-oxazolidinone and/or N,N'-bis (2-hydroxyethyl)urea may be contacted with the base for a period of between 10 minutes and 200 hours, preferably between 1 hour and 100 hours, although the contact time will depend upon such factors as the concentrations and types of reactants used.

The product N-(2-hydroxyethyl)-2-imidazolidinone may be purified by known processes, for example recrystallisation, and vacuum distillation.

N-(2-hydroxyethyl)-2-imidazolidinone prepared according to the process of the present invention may be used for the preparation of N-(vinyl)-2-imidazolidinone by dehydration by known methods. N-(2-hydroxyethyl)-2-imidazolidinone may also be used in the preparation of fire retardants for thermoplastic polymers as in EP 0196135.

The invention will now be illustrated by the following Examples.

In the Examples the following abbreviations have been used: MEA = monoethanolamine, having the formula:

$H_2N\text{-}CH_2\text{-}CH_2\text{-}OH$ (I)

BHEU = N,N'-bis(2-hydroxyethyl)urea, having the formula:

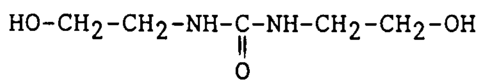

(II)

HEIDN = N-(2-hydroxyethyl)-2-imidazolidinone, having the formula:

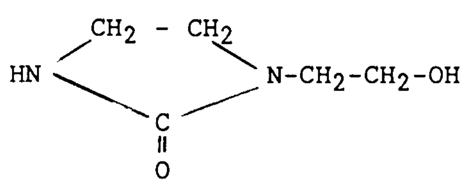

(III)

Adduct = the N-(2-hydroxyethyl)carbamate salt of monoethanolamine, having the formula:

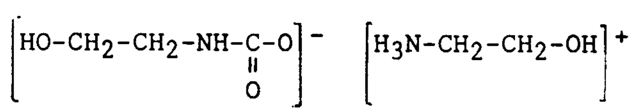

(IV)

2-oxazolidinone has the formula:

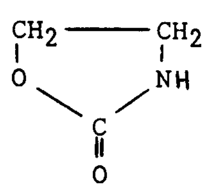

## Examples 1 to 5 - Preparation of Adduct and BHEU

A 70 ml Parr autoclave reactor fitted with a magnetic stirrer was used for reactions of monoethanolamine (MEA) and carbon dioxide to produce the adduct (monoethanolamine N-(2-hydroxyethyl) carbamate) and BHEU (N,N'-bis(2-hydroxyethyl)urea). The reactor was charged with MEA (about 4g, 65 mmol) (Aldrich 99%) and optionally potassium carbonate as 50% aqueous solution. The reactor was sealed and pressurised to 41.4 barg (600 psig) with carbon dioxide. Carbon dioxide was added to maintain the pressure until uptake had ceased. The reactor was then heated for the required time before cooling and depressurising. The products were removed directly or as methanol solutions and analysed by gas chromatography for MEA before being filtered and dried in vacuo (5 mm Hg and 40°C) and analysed by $^1H$ and $^{13}C$ NMR spectroscopy.

The results are shown in Table 1. Referring to Table 1 it will be seen that at temperatures up to 180°C in the absence of base catalyst and up to 130°C in the presence of base catalyst, monoethanolamine and carbon dioxide reacted to form the Adduct and BHEU.

Examples 6 to 9 - Preparation of HEIDN

The experimental procedure adopted for Examples 1 to 5 was repeated using potassium carbonate catalyst in a molar ratio of 1:10, carbonate : MEA, and at 180°C for reaction periods of 3 to 60 hours.

The results are shown in Table 1. Referring to Table 1 it will be seen that at 180°C in the presence of potassium carbonate, monoethanolamine and carbon dioxide reacted to produce HEIDN. In Example 8 after 12 hours at 180°C conversion of MEA was 86% and selectivity to HEIDN was 72% of theoretical, whereas in Example 5 in the absence of base only Adduct and BHEU were formed.

Example 10 - Preparation of Adduct

A 10 litre stainless steel Parr autoclave was used for the preparation of the Adduct. The autoclave was charged with MEA (2070g, 34 mol) and methanol solvent (270g). The autoclave was sealed and pressurised to about 41.4 barg (600 psig) with carbon dioxide at ambient temperature.

The MEA and carbon dioxide were allowed to react for about 20 minutes before the autoclave was repressurised with carbon dioxide. The process was repeated so that, in total, the autoclave had been pressurised five times and no more carbon dioxide was taken up. The autoclave was then repressurised to 41.4 barg and heated to 110°C for 60 hours without any further addition of carbon dioxide. After this period the reactor was cooled and depressurised and a viscous yellow liquid product (2915g) was recovered. The results are shown in Table 1.

Example 11 - Preparation of HEIDN

The Adduct product from Example 10 (2800g) was charged to the same 10 litre autoclave together with anhydrous potassium carbonate (350g, about 7 mol % ratio to adduct). The autoclave was pressurised to 10.3 barg (150 psig) with carbon dioxide and heated to 180°C for 24 hours. The initial pressure was 44.5 barg (645 psig), falling to 26.2 barg (380 psig) after 24 hours. The autoclave was cooled and depressurised and a red/brown liquid product with brown solid was recovered. The material was filtered to yield HEIDN product(3154g). The results are shown in Table 1.

Example 12 - Preparation of HEIDN from 2-oxazolidinone and MEA

A 70 ml Parr autoclave was charged with 2-oxazolidinone (6g, 69 mmol) (Aldrich, 98%), monoethanolamine (4g, 69 mmol) and anhydrous potassium carbonate (1g, 10 mol % ratio to MEA). The reactor was pressurised to 41.4 barg (600 psig) with nitrogen and heated to 180°C for 12 hours. The autoclave was then cooled and depressurised and the product recovered in methanol (30 ml). Removal of the solvent in vacuo produced a golden yellow viscous liquid (8.8g) which was analysed by $^{13}C$ NMR and $^1H$ NMR. The product yield was 85% by weight that is 85g of HEIDN for every 100g of 2-oxazolidinone charged.

Example 13 - Purification of HEIDN

A vacuum distillation of the product from Example 11 (74% HEIDN) produced a pale yellow product fraction boiling at about 230°C at 0.2 mm Hg pressure, containing 90% HEIDN as analysed by $^{13}C$ NMR. Redistillation of the pale yellow liquid did not improve its purity.

TABLE 1

| Example | $K_2CO_3$ (mol %) | Temp. (°C) | Time (h) | % conversion (1) | % selectivity (2) to | | |
|---|---|---|---|---|---|---|---|
| | | | | | Adduct | BHEU | HEIDN |
| 1 | none | 120 | 60 | 100 | 100 | - | - |
| 2 | 5 | 120 | 60 | 100 | 90 | 10 | - |
| 3 | 10 | 120 | 60 | 100 | 92 | 8 | - |
| 4 | 10 | 130 | 60 | 100 | 85 | 15 | - |
| 5 | none | 180 | 12 | 100 | 89 | 11 | - |
| 6 | 10 | 180 | 3 | 100 | 44 | 18 | 26 |
| 7 | 10 | 180 | 6 | 100 | 17 | 5 | 63 |
| 8 | 10 | 180 | 12 | 86 | - | - | 72 |
| 9 | 10 | 180 | 60 | 95 | - | - | 63 |
| 10* | none | 110 | 60 | 100 | 100 | - | - |
| 11* | 7 | 180 | 24 | 90 | - | - | 74 |

* Large scale experiment.

(1) % convertion with respect to MEA as determined by gc analysis for residual MEA.

(2) measured by $^1H$ NMR and expressed as a % of theoretical.

## Claims

1. A process for the production of N-(2-hydroxyethyl)-2-imidazolidinone which comprises contacting at elevated temperature, a base with one or more of:
   (a) monoethanolamine and carbon dioxide;
   (b) the N-(2-hydroxyethyl) carbamate salt of monoethanolamine;
   (c) 2-oxazolidinone and monoethanolamine; and
   (d) N,N'-bis (2-hydroxyethyl) urea.

2. A process as claimed in claim 1 in which the base comprises a carbonate, bicarbonate or hydroxide salt of an alkali metal or of an alkaline earth metal.

3. A process as claimed in claim 2 in which the base comprises potassium or sodium carbonate.

4. A process as claimed in any one of the preceding claims in which the carbamate salt is prepared by contacting monoethanolamine with carbon dioxide at a temperature of about 25 °C to 240 °C and a pressure of about atmospheric to 150 barg.

5. A process as claimed in any of the preceding claims in which the 2-oxazolidinone is prepared by reaction of monoethanolamine with carbon dioxide.

6. A process as claimed in any one of the preceding claims in which the N,N'-bis(2-hydroxyethyl) urea is prepared by the reaction of 2-oxazolidinone with monoethanolamine.

7. A process as claimed in claim 6 in which the 2-oxazolidinone for the preparation of N,N'-bis(2-hydroxyethyl)urea is prepared by reaction of monoethanolamine with carbon dioxide.

8. A process as claimed in any one of the preceding claims which is performed in the presence of carbon dioxide at a partial pressure in the range 20 to 60 barg.

European Patent Office

# EUROPEAN SEARCH REPORT

Application Number

EP 91 31 2022

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 183 076 (MITSUI TOATSU CHEMICALS, INC.)<br>--- | | C07D233/32<br>C08K5/3445 |
| A | EP-A-0 131 801 (BAYER AKTIENGESELLSCHAFT)<br>--- | | |
| A | EP-A-0 087 659 (BAYER AKTIENGESELLSCHAFT)<br>--- | | |
| A | EP-A-0 248 220 (MITSUI TOATSU CHEMICALS, INC.)<br>--- | | |
| A | EP-A-0 280 781 (HÜLS AKTIENGESELLSCHAFT)<br>--- | | |
| A | EP-A-0 285 500 (SOCIETE FRANÇAISE HOECHST SOCIETE ANONYME)<br>--- | | |
| D,A | FR-A-1 238 352 (SOCIETE DES USINES CHIMIQUES RHONE-POULENC)<br>--- | | |
| D,A | US-A-2 812 333 (UNION CARBIDE CORPORATION)<br>----- | | |
| | | | TECHNICAL FIELDS SEARCHED (Int. Cl.5) |
| | | | C07D<br>C08K |

The present search report has been drawn up for all claims

| Place of search | Date of completion of the search | Examiner |
|---|---|---|
| THE HAGUE | 04 MAY 1992 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)